Europäisches Patentamt

European Patent Office

Office européen des brevets

(11) Veröffentlichungsnummer: **0 133 669**
**B1**

(12) **EUROPÄISCHE PATENTSCHRIFT**

(45) Veröffentlichungstag der Patentschrift: **27.12.90**

(51) Int. Cl.⁵: **C 07 D 323/06**

(21) Anmeldenummer: **84108852.9**

(22) Anmeldetag: **26.07.84**

(54) **Verfahren zur Herstellung von Trioxan aus wässrigen, handelsüblichen Formaldehydlösungen.**

(30) Priorität: **04.08.83 DE 3328126**

(43) Veröffentlichungstag der Anmeldung:
**06.03.85 Patentblatt 85/10**

(45) Bekanntmachung des Hinweises auf die
Patenterteilung:
**27.12.90 Patentblatt 90/52**

(84) Benannte Vertragsstaaten:
**BE DE FR GB IT NL**

(56) Entgegenhaltungen:
**DE-A-1 543 340**
**DE-A-1 543 390**
**FR-A-1 429 161**
**GB-A-1 130 513**

**Die Akte enthält technische Angaben, die nach
dem Eingang der Anmeldung eingereicht
wurden und die nicht in dieser Patentschrift
enthalten sind.**

(73) Patentinhaber: **BASF Aktiengesellschaft
Carl-Bosch-Strasse 38
D-6700 Ludwigshafen (DE)**

(72) Erfinder: **Schmidt, Rudolf, Dr.
Paul-Klee-Strasse 1
D-6710 Frankenthal (DE)**
Erfinder: **Maurer, Gerd, Dr.
Im Emmertsgrund 64
D-6900 Heidelberg (DE)**
Erfinder: **Deck, Heribert
Josef-Huber-Strasse 3
D-6700 Ludwigshafen (DE)**

Courier Press, Leamington Spa, England.

## Beschreibung

Die vorliegende Erfindung betrifft ein Verfahren zur Herstellung von Trioxan aus wäßrigen, handelsüblichen Formaldehydlösungen.

Solche Verfahren sind beispielsweise in den Schriften DAS 15 43 340 und DOS 15 43 390 beschrieben. Bei diesen beschriebenen Verfahren werden konzentrierte Formaldehydlösungen — die in Trioxanlösungen umgesetzt werden — durch Destillation bei Normaldruck — häufig mittels Fallfilmverdampfer — gewonnen. Diese Verfahren sind jedoch energieaufwendig, da das Destillat überwiegend aus Wasser besteht. In der englischen Patentschrift 1 130 513 wird ein Verfahren beschrieben, bei dem der Nachteil des hohen Energieverbrauchs dadurch gemindert wird, daß handelsübliche, wäßrige Formaldehydlösungen mittels einer Druckdestillation aufkonzentriert werden, das Kopfprodukt Formaldehyd/Wasser in einem Dephlegmator partiell kondensiert wird, die Flüssigphase als Rücklauf in die Druckdestillation zurückgeführt wird und die Gasphase — angereichert mit Formaldehyd — als Wärmeträger und Reaktionsteilnehmer nach einer Entspannung auf ein Druckniveau unterhalb Atmosphärendruck einem Flüssigphasenreaktor zugeführt wird, in dem die Umsetzung zu Trioxan erfolgt. Mittels einer reinen der Reaktion nachgeschalteten Verstärkungskolonne, die bei demselben Druck wie der Flüssigphasenreaktor arbeitet, wird am Kopf dieser Verstärkungskolonne (Vakuumkolonne) ein Gemisch aus Trioxan, Formaldehyd und Wasser abgezogen, das nach bekannten Verfahren in seine Einzelkomponenten zerlegt wird. Das Sumpfprodukt der Verstärkungskolonne wird wiederum durch die Reaktionszone hindurch in die Druckdestillation zurückgeführt.

Bei dem zuletzt genannten Verfahren ist wirtschaftlich noch von Nachteil, daß einerseits aus der Reaktionszone saurer Katalysator in die Druckkolonne zurückgeführt wird, was zu Nebenreaktionen des Formaldehyds in der Druckkolonne führen kann, und daß andererseits mangels eines Abtreibsteils in der Vakuumkolonne Trioxan in die Druckkolonne zurückgeführt wird, was zu Ausbeuteverlusten führt.

Der Erfindung liegt daher die Aufgabe zugrunde, ein Verfahren zur Herstellung von Trioxan zu entwickeln, bei dem die Nachteile des bekannten Verfahrens vermieden werden können.

Diese Aufgabe wird bei einem Verfahren zur Herstellung von Trioxan aus wäßrigen, handelsüblichen Formaldehydlösungen in Gegenwart saurer Katalysatoren gelöst, wobei die Formaldehydlösungen (1) in eine oberhalb Atmosphärendruck betriebene Destillationskolonne (2) geleitet werden, im Sumpf der Destillationskolonne (2) Wasser (3) abgetrennt wird, und das an Formaldehyd angereicherte Kopfprodukt (4) in eine bei niedrigerem Druck als die Destillationskolonne (2) arbeitende Reaktionszone (5) entspannt wird, dadurch gekennzeichnet, daß die in der Reaktionszone (5) anfallenden Reaktionsprodukte (6) in eine unter niedrigerem Druck als die Reaktionszone (5) arbeitende zweite Destillationskolonne (7), bestehend aus Verstärkungs- und Abtreibsteil, geleitet werden, als Kopfprodukt (8) der zweiten Destillationskolonne (7) ein an Trioxan angereichertes Gemisch gewonnen wird, und als Sumpfprodukt (9) der zweiten Destillationskolonne (7) eine an Formaldehyd angereicherte Lösung als Rücklauf direkt unter Umgehung der Reaktionszone in die unter Druck arbeitende erste Destillationskolonne (2) zurückgeleitet wird.

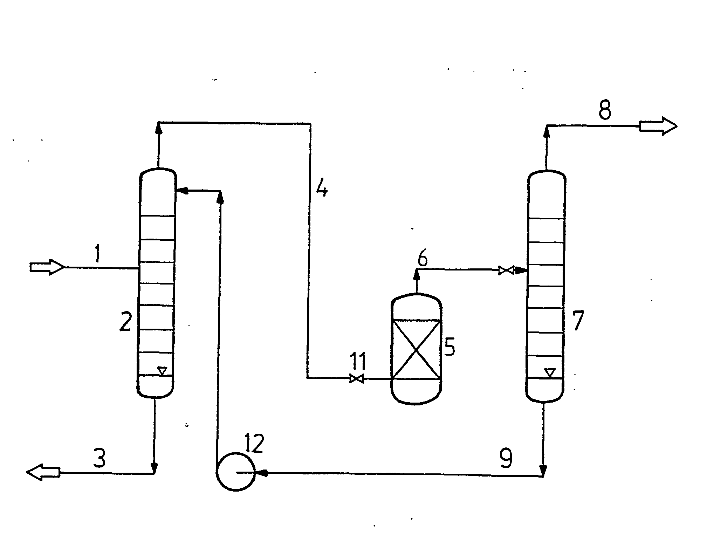

Ein Ausführungsbeispiel der Erfindung ist in der Zeichnung dargestellt und wird im folgenden näher beschrieben. Die Zeichnung zeigt ein schematisches Verfahrensfließbild des erfindungsgemäßen Verfahrens.

Das Ausgangsprodukt 1 — eine wäßrige, handelsübliche Formaldehydlösung — wird der oberhalb Atmosphärendruck betriebenen Destillationskolonne 2 zugeführt. Am Sumpf der Destillationskolonne wird Wasser 3 abgetrennt, als Kopfprodukt wird ein an Formaldehyd angereichertes Dampfgemisch 4 über ein Entspannungsventil 11 einem bei tieferem Druck als die Kolonne 2 arbeitenden Reaktor 5 zugeführt, in dem die teilweise Umsetzung des Formaldehyds zu Trioxan erfolgt. Das Reaktionsgemisch 6 — Formaldehyd/Trioxan/Wasser — gelangt anschließend in eine zweite, unter gleichem oder niedrigerem Druck wie der Reaktor arbeitende Destillationskolonne 7, bestehend aus Verstärkungs- und Abtreibsteil, aus der als Kopfprodukt 8 ein an Trioxan angereichertes Gemisch abgezogen wird, das nach bekannten Verfahren in seine Einzelkomponenten zerlegt wird, und als Sumpfprodukt 9 eine an Formaldehyd angereicherte Lösung gewonnen wird, die in den Reaktor 5 geleitet wird. Die für die Destillationskolonne 2 notwendige Rücklaufmenge 10 — mittels des Abtriebsteils der Destillationskolonne 7 angereichert mit Formaldehyd — wird mittels einer Pumpe 12 — notwendig für die Druckerhöhung — der Destillationskolonne 2 zugeführt.

## Patentanspruch

Verfahren zur Herstellung von Trioxan aus wäßrigen, handelsüblichen Formaldehydlösungen in Gegenwart saurer Katalysatoren, wobei die Formaldehydlösungen (1) in eine oberhalb Atmosphärendruck betriebene Destillationskolonne (2) geleitet werden, im Sumpf der Destillationskolonne (2) Wasser (3) abgetrennt wird, und das an Formaldehyd angereicherte Kopfprodukt (4) in eine bei niedrigerem Druck als die Destillationskolonne (2) arbeitende Reaktionszone (5) entspannt wird, dadurch gekennzeichnet, daß die in der Reaktionszone (5) anfallenden Reaktionsprodukte (6) in eine unter niedrigerem Druck als die Reaktionszone (5) arbeitende zweite Destillationskolonne (7), bestehend aus Verstärkungs- und Abtreibsteil, geleitet werden, als Kopfprodukt (8) der zweiten Destillationskolonne (7) ein an Trioxan angereichertes Gemisch gewonnen wird, und als Sumpfprodukt (9) der zweiten Destillationskolonne (7) eine an Formaldehyd angereicherte Lösung als Rücklauf direkt unter Umgehung der Reaktionszone in die unter Druck arbeitende erste Destillationskolonne (2) zurückgeleitet wird.

**Revendication**

Procédé de préparation de trioxane, à partir de solutions de formaldéhyde du commerce, aqueuses, les solutions de formaldéhyde (1) étant envoyées dans une colonne de distillation (2) opérant au-dessus de la pression atmosphérique, l'eau (3) étant séparée au fond de la colonne de distillation (2) et le produit de tête (4) enrichi en formaldéhyde étant détendu dans une zone de réaction (5) opérant à une pression inférieure à celle de la colonne de distillation (2), caractérisé par le fait que les produits de réaction (6) venant de la zone de réaction (5) sont envoyés dans une seconde colonne de distillation (7) opérant sous une pression inférieure à celle de la zone de réaction (5) et formées d'une partie concentration et d'une partie séparation, un mélange enrichi en trioxane est obtenu comme produit de tête (8) de la seconde colonne de distillation (7) et, comme produit de fond (9) de la seconde colonne de distillation (7), une solution enrichie en formaldéhyde est renvoyée, comme reflux, directement, en contournant la zone de réaction, dans la première colonne de distillation (2) opérant sous pression.

**Claim**

A process for the preparation of trioxane from a commercial aqueous formaldehyde solution in the presence of an acidic catalyst, in which said solution (1) is passed into a distillation column (2) operated under superatmospheric pressure, water (3) is separated off at the bottom of this column (2), and the top product (4), which is enriched in formaldehyde, is let down into a reaction zone (5) operated under lower pressure than the distillation column (2), wherein the products (6) obtained in the reaction zone (5) are passed into a second distillation column (7) comprising a rectifying section and stripping section, and operated under a pressure which is lower than that of the reaction zone (5), a mixture enriched in trioxane is obtained as the top product (8) of the second distillation column (7), and a solution enriched in formaldehyde is taken off as the bottom product (9) from the second distillation column (7) and fed, as a reflux, directly, circumventing the reaction zone, into the first distillation column (2) operated under super-atmospheric pressure.